# EUROPEAN PATENT SPECIFICATION

(11) **EP 4 182 376 B1**
(45) Date of publication and mention of the grant of the patent: **28.10.2026**
(21) Application number: 21752799.3
(22) Date of filing: 13.07.2021
(51) Int. Cl.: C08G 65/327, A61Q 17/04, C07F 9/09

(54) **POLYETHER PHOSPHATE ESTER COMPOUNDS, COMPOSITIONS AND USES**
POLYETHERPHOSPHATESTERVERBINDUNGEN, ZUSAMMENSETZUNGEN UND VERWENDUNGEN
COMPOSÉS D'ESTER DE PHOSPHATE DE POLYÉTHER, COMPOSITIONS ET UTILISATIONS

(30) Priority: 15.07.2020 US 202063052126 P
(43) Date of publication of application: 24.05.2023
(73) Proprietor: Croda, Inc., Plainsboro, NJ 08536 (US)
(72) Inventor: GUNDERMAN, Erik, Plainsboro, NJ 08536 (US); SAPORITO, Paul Joseph, Plainsboro, NJ 08536 (US); PEREIRA, Abel Goncalves, Plainsboro, NJ 08536 (US); DEXTER, Marni, Plainsboro, NJ 08536 (US); KAUR, Harpreet, Plainsboro, NJ 08536 (US)
(74) Representative: Pidgeon, George William
(86) International application number: PCT/US2021/041358
(87) International publication number: WO 2022/015681

(56) References cited:
- WO-A1-2020/066442
- DD-A1- 159 573
- JP-A- 2019 043 044
- JP-A- S4 920 153
- US-A1- 2006 088 582
- US-A1- 2007 048 241
- JADHAV SATISH ET AL: "Solid-Supported Porphyrins Useful for the Synthesis of Conjugates with Oligomeric Biomolecules", BIOCONJUGATE CHEMISTRY, vol. 27, no. 4, 20 April 2016 (2016-04-20), US, pages 1023 - 1029, XP055853117, ISSN: 1043-1802, DOI: 10.1021/acs.bioconjchem.6b00051

## Description

### Cross-Reference to Related Applications

This application claims priority to United States Provisional Application No. 63/052,126, titled "POLYETHER PHOSPHATE ESTER COMPOUNDS, COMPOSITIONS AND USES", filed July 15, 2020.

### Field of the Invention

The present invention relates to a compound which is a polyether phosphate ester, a composition comprising the compound and formulations, uses and methods comprising the compound or composition.

### Background

Alkoxylated phosphate esters have been used for many years as surface active agents (or surfactants), having emulsifying, dispersing, wetting and/or solubilising properties in a wide range of applications such as personal care, home care, health care and many others. In particular, alkoxylated phosphate esters have been used as emulsifiers in personal care applications, for example skin care, sunscreens, toiletries, decorative cosmetics, perfumes and fragrances.

Polyether phosphate esters are anionic surfactants which have previously been produced by the reaction of alkoxylates such as ethoxylated alcohols with polyphosphoric acid or phosphorous pentoxide. Ethoxylates are produced by the use of ethylene oxide. Such polyether phosphate esters have been used as surfactants in personal care applications and other applications. US2007/0048241A1 discloses an emulsifying system which is useful in products susceptible to phase inversion.

It is an object of the present invention to address at least one of the disadvantages associated with the prior art.

### Summary of the Invention

The present invention is based in part on the recognition that by the use of diols or glycols (such as propane diol) polyether phosphate esters can be made which avoid the use of epoxides or alkylene oxides (such as ethylene oxide or propylene oxide). The resulting compounds of the invention advantageously do not comprise any epoxide or alkylene oxide residues within the compound and there is also no residual unreacted alkylene oxide and/or alkylene oxide by-product in a composition comprising the compound since no alkylene oxide is used in the production process. Such compounds and compositions may also advantageously be effective surfactants in general and/or emulsifiers in particular. A specific example of a type of emulsion which a compound or composition according to the invention may be advantageous in making is a water-in-oil-in-water (W/O/W) emulsion.

A further advantage of avoiding the use of alkylene oxides in producing the compounds of the invention is that the majority of alkylene oxides are derived from petrochemical feedstocks. Thus a polyether phosphate ester which does not use an alkylene oxide in its production may have an improved environmental profile.

Viewed from a first aspect, the present invention provides a composition as defined in the claims.

Viewed from a second aspect, the present invention provides an emulsion as defined in the claims.

Viewed from a third aspect, the present invention provides a personal care formulation as defined in the claims.

Viewed from a fourth aspect, the present invention provides the use of the composition as defined in the claims.

Viewed from a fifth aspect, the present invention provides a method of forming an emulsion as defined in the claims.

Any or all of the features described herein may be combined in any aspect of the invention in any combination.

### Brief Description of the Drawings

FIG. 1 shows a microscope image of the emulsion produced when Product 1 (a composition according to the invention as made in Example 1) was used to emulsify mineral oil in water.
FIG. 2 shows a comparative emulsion to FIG. 1 in which Crodafos CES (a comparative phosphate ester emulsifier) was used to emulsify mineral oil in water.
FIG. 3 shows the emulsion when Product 1 was used to emulsify Isopropyl Myristate in water.
FIG. 4 shows a comparative emulsion to FIG. 3 in which Crodafos CES was used to emulsify Isopropyl Myristate in water.
FIG. 5 shows the emulsion when Product 1 was used to emulsify Di-isopropyl adipate in water.
FIG. 6 shows a comparative emulsion to FIG. 5 in which Crodafos CES was used to emulsify Di-isopropyl adipate in water.
FIG. 7 shows the size distribution (µm diameter) of mineral oil particles emulsified in water with Product 1.
FIG. 8 shows the size distribution (µm diameter) of mineral oil particles emulsified in water with Crodafos CES.

### Detailed Description of the Invention

It will be understood that any upper or lower quantity or range limit used herein may be independently combined.

It will be understood that, when describing the number of carbon atoms in a substituent group (*e.g.,* 'C1 to C6'), the number refers to the total number of carbon atoms present in the substituent group, including any present in any branched groups.

The term 'alkyl' when used herein means a hydrocarbyl group which is aliphatic, unsubstituted and saturated.

The term 'alkenyl' when used herein means a hydrocarbyl group which is aliphatic, unsubstituted and unsaturated.

The term 'end-cap' when used herein means a terminal group in a product which is unreactive. For example, if a diol reacts with a single mono-alcohol to form a product compound with one terminal hydroxyl group (reactive) and one terminal hydrocarbyl group (unreactive end cap), that would be referred to as a 'single end-capped' compound. If the diol reacted with two mono-alcohols to form a product compound with no terminal hydroxyl groups and two terminal hydrocarbyl groups (unreactive end caps), that would be referred to as a 'fully end-capped' compound.

The term 'residue' when used herein is the part of a reactant molecule which remains in the product compound after a reaction has occurred. For example, an alkylene oxide residue is the part of an alkylene oxide molecule which remains in a compound after an alkylene oxide reaction has occurred.

Many of the chemicals which may be used to produce the compound and composition of the present invention are obtained from natural sources. Such chemicals typically include a mixture of chemical species due to their natural origin. Due to the presence of such mixtures, various parameters defined herein can be an average value and may be non-integral.

### Polyether phosphate ester

The polyether phosphate ester does not comprise an alkylene oxide residue. By this it is understood that the polyether phosphate ester is not made with (or obtainable from) alkylene oxide reactants. Preferably the polyether phosphate ester does not comprise any alkylene oxide by-products. Preferably the polyether phosphate ester does not comprise an epoxide residue. Preferably the polyether phosphate ester does not comprise a propylene oxide residue or propylene oxide by-products. Preferably the polyether phosphate ester does not comprise an ethylene oxide residue or ethylene oxide by-products.

The use of alkylene oxide reactants to produce compounds may result in undesirable by-products or degradation products being formed. Dioxanes such as 1,4 dioxane can be an undesirable by-product or degradation product in compositions comprising compounds that are produced using ethylene oxide. This is especially true in phosphate esters that are made using an alkylene oxide because the low pH of the resulting compound makes the formation of undesirable by-products or degradation products more likely. Preferably the compound of the invention comprises no dioxanes, more preferably no 1,4 dioxane.

The polyether phosphate ester is preferably obtainable by phosphating a polyether which is produced by reacting a mono-alcohol and a diol to produce the polyether. The polyether may be a mixture of species. Preferably the polyether is the reaction product of reactants comprising a diol and a mono-alcohol.

The polyether preferably comprises at least 3 ether bonds, more preferably at least 4, particularly at least 5, desirably at least 6. The polyether may comprise at most 20 ether bonds, more preferably at most 15, particularly at most 10. Preferably the compound comprises 3 to 15 ether bonds.

The mono-alcohol reactant used to make the polyether may comprise at least 6 carbon atoms, preferably at least 8, desirably at least 10, especially at least 12. The mono-alcohol may comprise at most 36 carbon atoms, preferably at most 24 carbon atoms, more preferably at most 22, yet more preferably at most 20, particularly at most 18. Preferably, the mono-alcohol comprises 12 to 20 carbon atoms. The mono-alcohol may be a mixture of at least two mono-alcohols. Preferably, the mono-alcohol comprises C16 and C18 mono-alcohols.

The mono-alcohol is preferably a primary mono-alcohol. The mono-alcohol may be linear or branched, saturated or unsaturated. The mono-alcohol is preferably linear. The mono-alcohol is preferably saturated. The mono-alcohol may be a fatty alcohol. The mono-alcohol preferably comprises a hydrocarbyl group bonded to a hydroxyl group. The mono-alcohol preferably comprises an alkyl or alkenyl group, particularly alkyl group bonded to a hydroxyl group.

Suitable linear mono-alcohols may be selected from the group consisting of hexanol, heptanol, octanol, nonanol, decanol, undecanol, dodecanol, tridecanol, tetradecanol, pentadecanol, hexadecanol, heptadecanol, octadecanol, nonadecanol, eicosanol, heneicosanol, docosanol, tricosanol and tetracosanol.

Alternatively, the mono-alcohol may be a branched, preferably saturated alcohol. Suitable branched mono-alcohols include isopalmityl alcohol and/or isostearyl alcohol. The branched mono-alcohol may also be a Guerbet alcohol, i.e. an alcohol formed by the Guerbet reaction. The Guerbet reaction is an organic reaction converting a primary aliphatic alcohol into its β-alkylated dimer alcohol with the loss of one equivalent of water. Preferred Guerbet alcohols include hexyl decyl alcohol, octyl decyl alcohol and octyl dodecyl alcohol.

The diol reactant used to make the polyether may comprise at least 2 carbon atoms and preferably at least 3 carbon atoms. The diol may comprise at most 22 carbon atoms, preferably at most 12, more preferably at most 10, yet more preferably at most 6. Preferably the diol comprises 2 to 6 carbon atoms, more preferably 3 or 4 carbon atoms, particularly preferably the diol comprises 3 carbon atoms.

The diol may be primary or secondary. The diol is preferably primary. Preferably the diol comprises two primary hydroxyl groups. The diol may be linear, branched, or cyclic. The diol is preferably linear or branched, more preferably linear.

The diol is preferably a linear diol comprising 3 carbon atoms. The diol is preferably propanediol, more preferably 1,3 propanediol, particularly non-epoxide 1,3 propanediol. Non-epoxide 1,3-propanediol can be advantageously derived from a renewable source. 1,3 propanediol has two primary hydroxyl groups, and so a polyether product made with it is preferably linear, and may be more flexible and less viscous than a polyether made with 1,2 propanediol.

In contrast, 1,2 propanediol has secondary hydroxyl end groups, is branched and is derived from propylene oxide (an alkylene oxide) which is a reactive epoxide.

The diol may be a mixture of at least two diols. The diol may comprise less than 20 wt% of branched species on the basis of the total amount of diol reactant used, preferably less than 15 wt%, more preferably less than 10 wt%, yet more preferably less than 5 wt%, particularly less than 1 wt%. The diol may comprise less than 20 wt% of 1,2 propanediol on the basis of the total amount of diol reactant used, preferably less than 15 wt%, more preferably less than 10 wt%, yet more preferably less than 5 wt%, particularly less than 1 wt%. Preferably the diol does not comprise 1,2 propanediol. Preferably the diol does not comprise ethanediol.

The molar ratio of diol reactant to mono-alcohol reactant used to produce the polyether may be at least 2:1, preferably at least 3:1, more preferably at least 4:1. The molar ratio of diol reactant to mono-alcohol reactant used to produce the polyether may be at most 20:1, preferably at most 15:1, more preferably at most 12:1, particularly at most 10:1. Preferably the molar ratio of diol reactant to mono-alcohol reactant used to produce the polyether is from 2:1 to 20:1, preferably from 3:1 to 12:1, particularly from 4:1 to 10:1.

The mono-alcohol used to make the polyether may react to provide at least one terminal hydrocarbyl group (or 'end-cap') in the polyether. The terminal hydrocarbyl group may have any of the features of the mono-alcohol herein described. Preferably the terminal hydrocarbyl group is a C6 to C36, preferably C8 to C22, hydrocarbyl group. Preferably the hydrocarbyl group is an alkyl or alkenyl group, particularly an alkyl group. Preferably the mono-alcohol provides only one terminal hydrocarbyl group (a 'single end-cap') in the polyether.

A catalyst may be used in the reaction of the mono-alcohol and the diol to produce the polyether. The catalyst may be selected from suitable strong acid catalysts. The catalyst may be a monoprotic acid. The catalyst may be an organic acid. Preferably the catalyst is a sulfonic acid. Preferably the catalyst is selected from sulphuric acid and triflic acid (trifluoromethanesulfonic acid/TFMS). Preferably the catalyst is trifluoromethanesulfonic acid.

The polyether may comprise a mixture of species with at least one free hydroxyl group and species with no free hydroxyl groups. Some of the polyether species may comprise two terminal hydrocarbyl groups resulting in a polyether with no free hydroxyl groups. Such a polyether species may be referred to as a 'fully end-capped' material and may not take part in the subsequent phosphating reaction.

Preferably the polyether mixture is then phosphated to produce the polyether phosphate ester of the invention. Preferably the polyether phosphate ester is obtainable by reacting the polyether with a phosphorus oxide, preferably with phosphorous pentoxide.

The polyether phosphate ester preferably comprises at least one terminal C6 to C36, preferably C8 to C22, hydrocarbyl group. The terminal hydrocarbyl group may have any of the features of the mono-alcohol herein described. The hydrocarbyl group is preferably an alkyl or alkenyl group. Preferably the polyether phosphate ester does not comprise a carboxylic acid ester bond.

The polyether phosphate ester may comprise mono-ester (comprising one phosphate ester group) and di-ester (comprising two phosphate ester groups). Preferably the amount of mono-ester is at least 10% of the total weight of phosphate ester, more preferably at least 25%, particularly at least 50%. Preferably the amount of mono-ester is at most 90% of the total weight of phosphate ester, more preferably at most 75%, particularly at most 50%. Preferably the amount of di-ester is at least 10% of the total weight of phosphate ester, more preferably at least 25%, particularly at least 50%. Preferably the amount of di-ester is at most 90% of the total weight of phosphate ester, more preferably at most 75%, particularly at most 50%.

### Composition of the Invention

The composition of the invention comprises:
i) a polyether phosphate ester which does not comprise an alkylene oxide residue;
ii) a mono-alcohol phosphate ester; and
iii) a polyether which comprises at least two terminal C6 to C36 hydrocarbyl groups.

Dioxanes such as 1,4 dioxane may be an undesirable by-product or degradation product in compositions comprising compounds that are produced using ethylene oxide. The composition of the invention advantageously comprise no dioxane. The composition of the invention is advantageously made without using ethylene oxide. The composition may comprise an undetectable amount of dioxane. Preferably the composition comprises less than 1 ppm dioxane, more preferably less than 0.5 ppm dioxane, yet more preferably less than 100 ppb dioxane, particularly less than 10 ppb dioxane, especially less than 1 ppb dioxane. The composition of the invention preferably comprises no alkylene oxide by-products or degradation products.

Preferably the polyether phosphate ester is a compound according to the first aspect of the invention. The polyether phosphate ester may comprise any of the features described herein with regard to the compound of the invention. The polyether phosphate ester is preferably obtainable by phosphating a polyether which is produced by reacting a mono-alcohol and a diol to produce the polyether. The polyether may be a mixture of species.

The polyether phosphate ester may comprise mono-ester (comprising one phosphate ester group) and di-ester (comprising two phosphate ester groups). Preferably the amount of mono-ester in the composition is at least 10% of the total weight of phosphate ester in the composition, more preferably at least 25%, particularly at least 50%. Preferably the amount of mono-ester in the composition is at most 90% of the total weight of phosphate ester in the composition, more preferably at most 75%, particularly at most 50%. Preferably the amount of di-ester in the composition is at least 10% of the total weight of phosphate ester in the composition, more preferably at least 25%, particularly at least 50%. Preferably the amount of di-ester in the composition is at most 90% of the total weight of phosphate ester in the composition, more preferably at most 75%, particularly at most 50%.

The mono-alcohol phosphate ester is preferably obtainable by reacting a mono-alcohol with a phosphorus oxide, preferably with phosphorous pentoxide. The mono-alcohol may comprise at most 36 carbon atoms, preferably at most 24 carbon atoms, more preferably at most 22, yet more preferably at most 20, particularly at most 18. Preferably, the mono-alcohol comprises from 12 to 18 carbon atoms. The mono-alcohol may be a mixture of at least two mono-alcohols. Preferably, the mono-alcohol comprises C16 and C18 mono-alcohols. The mono-alcohol is preferably a primary mono-alcohol. The mono-alcohol may be linear or branched, saturated or unsaturated. The mono-alcohol is preferably linear. The mono-alcohol is preferably saturated. The mono-alcohol may be a fatty alcohol. The mono-alcohol preferably comprises a hydrocarbyl group bonded to a hydroxyl group. The mono-alcohol preferably comprises an alkyl or alkenyl group, particularly alkyl group bonded to a hydroxyl group.

The polyether comprises at least two terminal C6 to C36 hydrocarbyl groups. Preferably the hydrocarbyl groups are alkyl or alkenyl groups. Preferably the polyether has no free hydroxyl groups, particularly the polyether is a fully end-capped polyether. The polyether preferably comprises at least 3 ether bonds, more preferably at least 4, particularly at least 5, desirably at least 6. The polyether may comprise at most 20 ether bonds, more preferably at most 15, particularly at most 10. The polyether is preferably obtainable by reacting a mono-alcohol and a diol. The mono-alcohol may have any of the features of a mono-alcohol described herein. The diol may have any of the features of a diol described herein.

Preferably the amount of polyether phosphate ester in the composition is at least 20 wt% on the basis of the total weight of the composition, more preferably at least 30 wt%, particularly at least 40 wt%, desirably at least 50 wt%. Preferably the amount of polyether phosphate ester in the composition is at most 90 wt%, more preferably at most 75 wt%, particularly at most 60 wt%.

Preferably the amount of mono-alcohol phosphate ester in the composition is at least 10 wt% on the basis of the total weight of the composition, more preferably at least 20 wt%, particularly at least 30 wt%. Preferably the amount of polyether phosphate ester in the composition is at most 90 wt%, more preferably at most 75 wt%, particularly at most 50 wt%.

Preferably the amount of polyether in the composition is at least 2 wt% on the basis of the total weight of the composition, more preferably at least 5 wt%, particularly at least 10 wt%. Preferably the amount of second polyether in the composition is at most 20 wt%, more preferably at most 15 wt%, particularly at most 10 wt%.

Preferably the weight ratio of polyether phosphate ester to mono-alcohol phosphate ester in the composition is at least 1.2:1, more preferably at least 1.5:1. This weight ratio may be at most 10:1, preferably at most 8:1.

Preferably the weight ratio of polyether phosphate ester to polyether in the composition is at least 2:1, more preferably at least 3:1, particularly at least 4:1. This weight ratio may be at most 20:1, preferably at most 15:1.

The composition may further comprise:
iv) unreacted mono-alcohol.

Without being bound by theory, including unreacted mono-alcohol in the composition may advantageously improve the processing characteristics of the composition, such as melting point and/or viscosity. Preferably, including unreacted mono-alcohol in the composition reduces the melting point of the composition. Preferably, including unreacted mono-alcohol in the composition reduces the kinematic viscosity of the composition at 25 °C.

The mono-alcohol may comprise at least 6 carbon atoms, preferably at least 8, desirably at least 10, especially at least 12. The mono-alcohol may comprise at most 36 carbon atoms, preferably at most 24 carbon atoms, more preferably at most 22, yet more preferably at most 20, particularly at most 18. Preferably, the mono-alcohol comprises from 12 to 18 carbon atoms. The mono-alcohol may be a mixture of at least two mono-alcohols. Preferably, the mono-alcohol comprises C16 and C18 mono-alcohols. The mono-alcohol is preferably a primary mono-alcohol. The mono-alcohol may be linear or branched, saturated or unsaturated. The mono-alcohol is preferably linear. The mono-alcohol is preferably saturated. The mono-alcohol may be a fatty alcohol.

Preferably the amount of unreacted mono-alcohol in the composition is at least 10 wt% on the basis of the total weight of the composition, more preferably at least 25 wt%, particularly at least 40 wt%. Preferably the amount of mono-alcohol in the composition is at most 90 wt%, more preferably at most 75 wt%, particularly at most 50 wt%.

The composition of the invention may be used as a surfactant, emulsifier, dispersant, stabiliser, solubiliser, pigment wetter, viscosity stabilizer and/or rheology modifier. The invention also includes the use of the composition as a surfactant, emulsifier, dispersant, stabilizer, solubiliser, pigment wetter, viscosity stabilizer and/or rheology modifier, preferably use as a surfactant and/or emulsifier, more preferably as an emulsifier.

### Emulsion

An emulsion according to the invention comprises the composition of the invention.

The compound or composition of the invention is suitable for use in producing emulsions (and dispersions), i.e. as the emulsifier, or as part of an emulsifier system. The emulsion may be a water-in-oil (W/O) emulsion, oil-in-polyol (e.g. glycerol) emulsion or oil-in-water (O/W) emulsion. The emulsion may be a multiple emulsion, for example a water-in-oil-in-water (W/O/W) emulsion. Preferably, the emulsion is a W/O/W emulsion.

The emulsion may comprise a dispersed phase having a D(v,0.1) particle size of less than 20 µm, preferably less than 15 µm. The D(v,0.1) value is the equivalent spherical diameter which 10% of all the particles (on a volume basis) fall below. Preferably the particle size is measured by laser light scattering, particularly as described herein.

The emulsion is preferably for use in a personal care formulation, more preferably a skin care, hair care, hair color, sunscreen, cosmetic, antiperspirant or dermatological product, particularly a skin care, hair care, hair color, sunscreen or antiperspirant product.

The oil phase of the emulsion preferably comprises an emollient oil of the type used in a personal care formulation. The emollient is preferably an oily material which is liquid at ambient temperature (i.e. about 23°C). Alternatively it can be solid at ambient temperature, in which case in bulk it will usually be a waxy solid, provided it is liquid at an elevated temperature at which it can be included in and emulsified in the composition.

The oil phase of the emulsion may comprise one or more of mineral oil, paraffin oil, ester oil, vegetable oil, silicon oil, alcohol or silicone.

Suitable oil phase components include non-polar oils, for example mineral or paraffin, especially isoparaffin, oils, such as that sold by Croda as Arlamol^{™} HD; or medium polarity oils, for example vegetable ester oils such as jojoba oil, vegetable glyceride oils, animal glyceride oils, such as that sold by Croda as Crodamol^{™} GTCC (caprylic/capric triglyceride), synthetic oils, for example synthetic ester oils, such as isopropyl palmitate and those sold by Croda as Crodamol IPP and Arlamol DOA, ether oils, particularly of two fatty e.g. C8 to C18 alkyl residues, such as that sold by Croda as Arlamol LFE (dicaprylether), guerbet alcohols such as that sold by Cognis as Eutanol G (octyl dodecanol), or silicone oils, such as dimethicone oil such as those sold by Dow Corning as Xiameter PMX-200, cyclomethicone oil, or silicones having polyoxyalkylene side chains to improve their hydrophilicity; or highly polar oils including alkoxylate emollients for example fatty alcohol propoxylates such as that sold by Croda as Arlamol PS15E (propoxylated stearyl alcohol). Suitable emollient materials that can be solid at ambient temperature but liquid at temperatures typically used to make the formulations of this invention include jojoba wax, tallow and coconut wax/oil.

Mixtures of emollients can and often will be used, and in some cases solid emollients may dissolve wholly or partly in liquid emollients or in combination the freezing point of the mixture is suitably low. Where the emollient composition is a solid (such as fatty alcohols) at ambient temperature, the resulting dispersion may technically not be an emulsion (although in most cases the precise phase of the oily disperse phase cannot readily be determined) but such dispersions behave as if they were true emulsions and the term emulsion is used herein to include such compositions.

The concentration of the oil phase may vary widely. The amount of oil in the emulsion is suitably in the range from 1 to 90%, preferably 3 to 60%, more preferably 5 to 40%, particularly 8 to 20%, and especially 10 to 15% by weight of the total formulation.

The concentration of aqueous phase (e.g. water or polyol, for example glycerin) present in the emulsion is suitably greater than 5%, preferably in the range from 30 to 90%, more preferably 50 to 90%, particularly 70 to 85%, and especially 75 to 80% by weight of the total formulation.

The amount of the composition of the invention in an emulsion or personal care formulation according to the present invention may be at least 0.1%, preferably at least 0.5%, more preferably at least 1%, by weight of the total formulation.

The amount of composition of the invention in an emulsion or personal care formulation according to the present invention may be at most 20%, preferably at most 15%, more preferably at most 10%, particularly preferably at most 6%, and especially preferably at most 5.5%, by weight of the total formulation.

The amount of composition of the invention in an emulsion or personal care formulation according to the present invention is suitably in the range from 0.1 to 10%, preferably 0.5 to 8%, more preferably 1 to 7%, particularly 1 to 6%, and especially 1 to 5.5%, by weight of the total formulation.

The emulsions according to the present invention may also contain other additional surfactant materials which form part of the emulsifier system. Other suitable surfactants include relatively hydrophilic surfactants, e.g. having a HLB value of greater than 10, preferably greater than 12, and relatively hydrophobic surfactants e.g. having a HLB value of less than 10, preferably less than 8. Relatively hydrophilic surfactants include alkoxylate surfactants with an average in the range from about 10 to about 100 alkylene oxide, particularly ethylene oxide residues; and relatively hydrophobic surfactants include alkoxylate surfactants preferably with an average in the range from about 3 to about 10 alkylene oxide, particularly ethylene oxide residues.

Personal care formulations according to the invention can be divided by viscosity into milks and lotions, which preferably have a low shear viscosity (measured at shear rates of about 0.1 to 10 s⁻¹ as is typically used in Brookfield viscometers) of up to 10,000 mPa.s, and creams which preferably have a low shear viscosity of more than 10,000 mPa.s. Milks and lotions preferably have a low shear viscosity in the range from 100 to 10,000, more preferably 200 to 5,000, and particularly 300 to 1,000 mPa.s. The amount of composition according to the present invention present in a milk or lotion is preferably in the range from 0.5 to 3% by weight of the total formulation.

Creams preferably have a low shear viscosity of at least 20,000, more preferably in the range from 30,000 to 80,000, and particularly 40,000 to 70,000 mPa.s, although even higher viscosities e.g. up to about 10⁶ mPa.s, may also be used. The amount of composition according to the present invention in a cream is preferably in the range from 1 to 5% by weight of the total formulation.

The emulsions of the invention may be made by generally conventional emulsification and mixing methods. For example, the composition of the invention may be added to (i) the oil phase, after which the aqueous phase is then added to the oil phase, or (ii) both the combined oil and water phases, or (iii) the water phase, which is then added to the oil phase. Method (i) is preferred. In all of these methods, the resulting mixture can then be emulsified using standard techniques. It is preferred to either heat the aqueous and oil phases usually above about 60°C, e.g., to about 80 to 85°C, or to subject the aqueous phase to high intensity mixing at lower, e.g., about ambient, temperature (cold process). Vigorous mixing and the use of moderately elevated temperatures can be combined if desired. The heating and/or high intensity mixing can be carried out before, during or after addition of the water to the oil phase.

The emulsions can also be made by inverse emulsification methods, whereby the composition of the invention is added to either the oil phase or the aqueous phase, and the aqueous phase is mixed into the oil phase to initially form a water in oil emulsion. Aqueous phase addition is continued until the system inverts to form an oil in water emulsion. Plainly a substantial amount of aqueous phase will generally be needed to effect inversion and so this method is not likely to be used for high oil phase content emulsions. Vigorous mixing and the use of moderately elevated temperatures can be combined if desired. Heating can be carried out during or after addition of the aqueous phase and before, during or after inversion. High intensity mixing can be carried out during or after addition of the aqueous phase, and before or during inversion.

The emulsions may for example be microemulsions or nanoemulsions, having a mean droplet size over a wide range, preferably in the range from 10 to 10,000 nm. In one embodiment, the emulsion droplet size may be reduced, for example by high pressure homogenisation, preferably to a value in the range from 100 to 1,000 nm, more preferably 300 to 600 nm.

The emulsions according to the present invention are stable, preferably for greater than one month, more preferably greater than two months, particularly greater than three months, at room temperature (i.e. about 20 °C). The stability at higher temperatures can be particularly important, and therefore the emulsion is stable suitably for greater than one week, preferably greater than two weeks, more preferably greater than 3 weeks, particularly greater than one month, at 50°C.

### Personal Care formulations

A personal care formulation according to the invention comprises a compound which is a polyether phosphate ester and which does not comprise an alkylene oxide residue, or comprises the composition of the invention.

Many other components may be included in the formulation to make a personal care or cosmetic formulation or product. These components can be oil soluble, water soluble or non-soluble. Examples of such materials include:
(i) preservatives such as those based on potassium sorbate, sodium benzoate, parabens (alkyl esters of 4-hydroxybenzoic acid), phenoxyethanol, substituted ureas and hydantoin derivatives e.g. those sold commercially under the trade names Germaben II Nipaguard BPX and Nipaguard DMDMH. Such preservatives are used preferably at a concentration in the range from 0.5 to 2% by weight of the total composition. A preservative booster such as caprylyl glycol or ethylhexylglycerin may also be used;
(ii) perfumes, when used preferably at a concentration in the range from 0.1 to 10% more preferably up to about 5%, and particularly up to about 2% by weight of the total composition;
(iii) humectants or solvents such as alcohols, polyols such as glycerol and polyethylene glycols, when used preferably at a concentration in the range from 1 to 10% by weight of the total composition;
(iv) sunfilter or sunscreen materials including organic sunscreens and/or inorganic sunscreens including those based on titanium dioxide or zinc oxide; when used preferably at a concentration in the range from 0.1% to 20%, more preferably 1 to 15%, and particularly 2 to 10% by weight of the total composition;
(v) alpha hydroxy acids such as glycolic, citric, lactic, malic, tartaric acids and their esters; self-tanning agents such as dihydroxyacetone;
(vi) antimicrobial, particularly anti-acne components such as salicylic acid;
(vii) vitamins and their precursors including: (a) Vitamin A, *e.g.,* as retinyl palmitate and other tretinoin precursor molecules, (b) Vitamin B, *e.g.,* as panthenol and its derivatives, (c) Vitamin C, *e.g.,* as ascorbic acid and its derivatives, (d) Vitamin E, *e.g.,* as tocopheryl acetate, (e) Vitamin F, *e.g.,* as polyunsaturated fatty acid esters such as gamma-linolenic acid esters;
(viii) skin care agents such as ceramides either as natural materials or functional mimics of natural ceramides;
(ix) phospholipids, such as synthetic phospholipids or natural phospholipids, *e.g.,* lecithin;
(x) vesicle-containing formulations;
(xi) germanium-containing compounds;
(xii) botanical extracts with beneficial skin care properties;
(xiii) skin whiteners such as Arlatone Dioic DCA^{™} sold by Croda, kojic acid, arbutin and similar materials;
(xiv) skin repair compounds actives such as Allantoin and similar series;
(xv) caffeine and similar compounds;
(xvi) cooling additives such as menthol or camphor;
(xvii) insect repellents such as N,N-diethyl-3-methylbenzamide (DEET) and citrus or eucalyptus oils;
(xviii) essential oils;
(xix) ethanol;
(xx) pigments, including microfine pigments, particularly oxides and silicates, e.g. iron oxide, particularly coated iron oxides, and/or titanium dioxide, and ceramic materials such as boron nitride;
(xxi) other solid components, such as are used in makeup and cosmetics, to give suspoemulsions, preferably used in an amount in the range from 1 to 15 wt%, more preferably from 5 to 15 wt% based on the total weight of the formulation; and
(xxii) deodorant or antiperspirant compounds.

The composition and emulsions according to the present invention are suitable for use in a wide range of personal care formulations and end-use applications, such as moisturizers, sunscreens, after sun products, body butters, gel creams, high perfume containing products, perfume creams, baby care products, hair treatments, hair colourants, shampoos, hair conditioners, skin toning and skin whitening products, water-free products, anti-perspirant and deodorant products, tanning products, cleansers, 2-in-1 foaming emulsions, multiple emulsions, preservative free products, mild formulations, scrub formulations e.g., containing solid beads, silicone in water formulations, pigment containing products, sprayable emulsions, cosmetics, colour cosmetics, conditioners, shower products, foaming emulsions, make-up remover, eye make-up remover, and wipes.

The formulation may be a spray, lotion, cream or ointment. When the formulation is a colour cosmetic, it may be a foundation, mascara, eyeshadow or lipstick. The formulation may be an anti-perspirant or deodorant. Preferably the formulation is a sunscreen.

The formulation may further comprise a sunfilter or sunscreen material, preferably a UV filter. The sunfilter or sunscreen material may be selected from organic sunscreens and inorganic sunscreens, preferably selected from inorganic sunscreens. The sunfilter or sunscreen material may be selected from titanium dioxide and zinc oxide. The compound or composition of the invention may advantageously improve the SPF of a sunscreen formulation as shown in the Examples.

Formulations containing a composition or emulsion according to the present invention may have a pH value over a wide range, preferably in the range from 3 to 13, more preferably 4 to 10, and especially 5 to 8.

### Applications outside personal care

The composition of the invention may have application outside of personal care.

A pharmaceutical or therapeutic formulation may comprise the composition of the invention.

A hard surface cleaner may comprise the composition of the invention. The hard surface cleaner may be an alkaline cleaner, oven cleaner or floor cleaner/stripper.

A laundry detergent may comprise the composition of the invention. The laundry detergent may be a spray dried, powder blended or liquid laundry detergents.

A textile or leather processing formulation may comprise the composition of the invention.

A rinse aid formulation for automated dish and glass washing systems may comprise the composition of the invention.

An agrochemical formulation may comprise the composition of the invention. Many agricultural adjuncts such as herbicides are required in aqueous solution for application to foliage. The composition of the invention may enable emulsification and/or solubilisation of additives into water together with good wetting to ensure optimum spreading onto a leaf's surface.

An oilfield chemical or well drilling formulation may comprise the compound or composition of the invention.

An emulsion polymerisation system may comprise the compound or composition of the invention.

An emulsion explosive system may comprise the compound or composition of the invention.

### Use as a surfactant/emulsifier

Viewed from a further aspect, the invention provides the use of the composition of the invention as a surfactant. The composition of the invention may be used as an emulsifier.

The composition of the invention may be used as an emulsifier in the production of a water-in-oil-in-water (W/O/W) emulsion, preferably in the production of a W/O/W emulsion by a method comprising a single emulsifying step. The compound or the composition of the invention may be used as the only emulsifier in the production of a water-in-oil-in-water (W/O/W) emulsion.

Preferably the invention provides the use of the composition of the invention to reduce the D(v,0.1) particle size of an emulsion. The compound or the composition of the invention may be used to prepare an emulsion with a dispersed phase having a D(v,0.1) particle size of less than 20 µm, preferably less than 15 µm. Preferably the emulsion is a personal care formulation. Preferably the particle size is measured by laser light scattering, particularly as described herein.

### Method of forming an emulsion

Viewed from a further aspect, the invention provides a method of forming an emulsion in which:
a) the composition of the invention;
b) a first phase component; and
c) a second phase component;
are combined in any order, including simultaneously.

For example, the composition of the invention may be added to (i) an oil phase, after which an aqueous phase is then added to the oil phase, or (ii) both the combined oil and water phases, or (iii) the water phase, which is then added to the oil phase. Method (i) is preferred. In all of these methods, the resulting mixture can then be emulsified using standard techniques. It is preferred to either heat the aqueous and oil phases usually above about 60°C, *e.g.,* to about 80 to 85°C, or to subject the aqueous phase to high intensity mixing at lower, e.g. about ambient, temperature (cold process). Vigorous mixing and the use of moderately elevated temperatures can be combined if desired. The heating and/or high intensity mixing can be carried out before, during or after addition of the water to the oil phase.

The emulsions can also be made by inverse emulsification methods, whereby the composition of the invention is added to either the oil phase or the aqueous phase, and the aqueous phase is mixed into the oil phase to initially form a water in oil emulsion. Aqueous phase addition is continued until the system inverts to form an oil in water emulsion. Plainly a substantial amount of aqueous phase will generally be needed to effect inversion and so this method is not likely to be used for high oil phase content emulsions. Vigorous mixing and the use of moderately elevated temperatures can be combined if desired. Heating can be carried out during or after addition of the aqueous phase and before, during or after inversion. High intensity mixing can be carried out during or after addition of the aqueous phase, and before or during inversion

Preferably the emulsion is a water-in-oil-in-water (W/O/W) emulsion.

Any or all of the features described herein may be combined in any aspect of the invention in any combination.

### Examples

The invention is illustrated by the following non-limiting examples. All parts and percentages are given by weight unless otherwise stated. All tests and physical properties herein have been determined at atmospheric pressure and room temperature (i.e. about 20 °C), unless otherwise stated herein, or unless otherwise stated in the referenced test methods and procedures.

### Test Methods

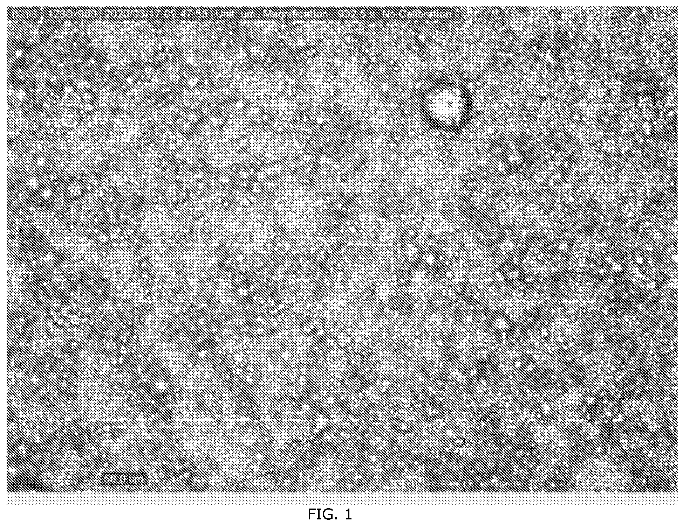

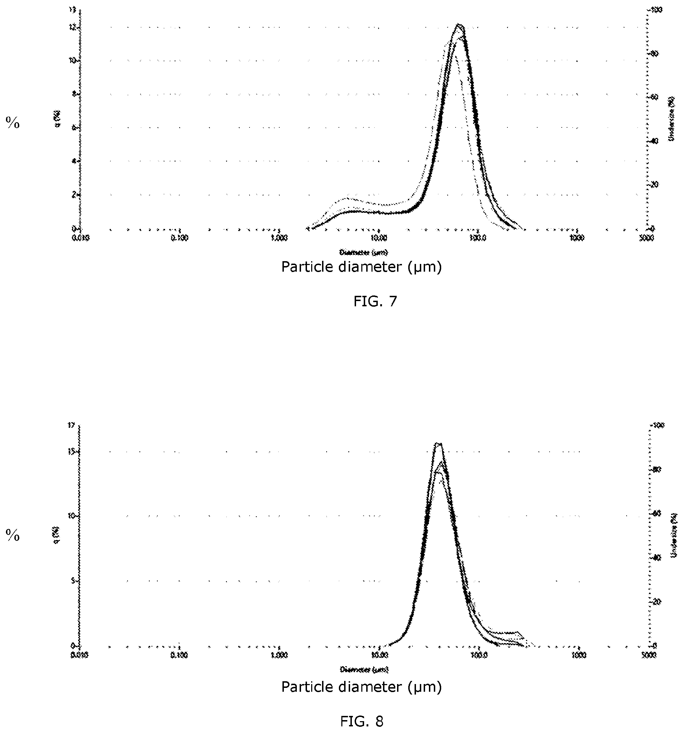

In this specification the following test methods have been used:
(i) The hydroxyl value is defined as the number of mg of potassium hydroxide equivalent to the hydroxyl content of 1 g of sample, and was measured by acetylation followed by hydrolysation of excess acetic anhydride. The acetic acid formed was subsequently titrated with an ethanolic potassium hydroxide solution.
(ii) The NMR analysis was conducted using Bruker equipment and TopSpin 3.2 processing software. 1H, 13C, and 31P NMR spectra (400 MHz) were obtained from the samples in deuterated acetone at 55 °C.
(iii) The microscope images shown in FIGS. 1 to 6 were taken using a Dino-Lite Edge 700-900x 5MP digital microscope. The emulsions were made under the same conditions (i.e. mixing speed and time). The microscope images were taken 24 hours after the emulsions were made at a magnification of 933x.
(iv) The particle size distributions shown in FIGS. 7 and 8 were produced using a Horiba LA-960 laser light scattering particle size analyser. The measurement method was:
   - Two pre-dispersions in deionized water were prepared of each sample
   - The pre-dispersions were mixed gently by inversion
   - Measurements were made of three aliquots of each of the pre-dispersions
   - The circulator was turned off during measurement to minimize potential bubble formation.
(v) The in-vitro SPF results in Example 6 were produced using a Labsphere UV-2000S, Solar light (16S-300-009s) and PMMA plates. The measurement method was:
   - 0.03g of formulation was applied on the rough side of the PMMA plate by adding dabs all over. In a circular motion, spread the formulation on the plate horizontally back and forth for even coverage. The treated plate was then placed in a dark drawer to dry for 15 minutes.
   - After 15 minutes, initial SPF readings were taken and then the plate was placed under the solar light simulator for a calculated time to be equal to 4MED exposures.
   - 5 readings per plate were taken and 3 plates were run per product according to regulations which require 15 data points
(vi) The actives delivery performance results in Example 7 were produced using a Diffusion Cells system (Perme Gear) also known as a Vertical Franz Cell and water bath circulator. The measurement method was:
   - Each Franz cell was filled with phosphate buffer saline (PBS; pH 7.4). A small stir bar was added, and the buffer was allowed to equilibrate.
   - Strat-M Membrane was soaked in PBS buffer for a few hours before the test placed on receptor chamber avoiding bubbles.
   - The donor compartment was clamped to the receptor compartment
   - Approximately 1 ml of formulation was applied into donor compartment
   - Samples were collected at various time intervals after 4, 8, and 24 hours
   - The samples were analyzed using Cary UV-Vis 60 between 200-400 nm wavelength to measure the weight % of active (caffeine) transferred.

### Example 1

Mixed C16/C18 fatty mono-alcohol (Crodacol 1618 ex Croda) is reacted with 1,3 propanediol at a molar ratio of 1:8 using triflic acid as the catalyst. The mixture is stirred and heated to around 180 °C for several hours until the hydroxyl value is in the range 60 to 90 mg KOH / g to produce a polyether intermediate material. The intermediate material is a mixture of hydroxyl functional polyether and fully end-capped polyether (i.e. the fully end-capped polyether comprises two terminal C16/C18 hydrocarbyl groups and has no free hydroxyl groups). This intermediate material is then neutralized and filtered. The intermediate material is charged into a reactor along with an equal amount, by weight, of mixed C16/C18 mono-alcohol and reacted with phosphorus pentoxide.

Once the phosphating reaction is complete the resulting active product comprises i) polyether phosphate ester, ii) mono-alcohol phosphate ester and iii) end-capped polyether. This active product is blended with mixed C16/C18 mono-alcohol to achieve a final product comprising 25 wt% mono-alcohol and 75 wt% active product. This final product composition according to the invention will be referred to as Product 1.

### Example 2

451.75 grams of 1,3 propanediol was charged to a glass reaction vessel equipped with a stirrer, thermometer, condenser and nitrogen sparge. With the temperature at 60-70°C., 198.25 grams of mixed C16/C18 fatty alcohol, 1.30 grams of trifluoromethanesulfonic acid, were charged to the glass reaction vessel. The batch was reacted at 150-180°C. until the hydroxyl value indicated that 5 moles of 1,3 propanediol had been reacted with 1 mole of mixed C16/C18 fatty alcohol. 245.52 grams of the resulting polyether preparation and 245.52 grams of mixed C16/C18 fatty alcohol were charged into a glass reaction vessel equipped with a stirrer, thermometer, condenser, and nitrogen sparge. The temperature of the vessel was increased to 70°C. 58.86 grams of phosphorous pentoxide was charged to the vessel over the course of 2 hours. The mixture was allowed to stir for 4 hours. This final product composition according to the invention will be referred to as Product 2.

### Example 3

Two samples of the active product from Example 1 (*i.e.,* the active product as described prior to the final blending step with mixed C16/C18 mono-alcohol to form Product 1) were analysed using NMR as described in the Test Methods above to determine the distribution of component species in the sample. The results of the NMR analysis are shown in Table 1.

**Table 1 - Phosphate ester distribution:**

| | **Component species** | **Sample A (wt%)** | | **Sample B (wt%)** | |
|---|---|---|---|---|---|
| | Phosphoric acid (H₃PO₄) | | 0.2% | | 0.2% |
| | Mono-phosphate ester | | 27.5% | | 25.9% |
| | Di-phosphate ester | | 35.5% | | 41.3% |
| | Unreacted C16/C18 alcohol | | 9.1% | | 8.3% |
| | Polyether species | | 27.7% | | 24.3% |
| | Total | | 100% | | 100% |

The distribution in Table 1 was calculated using idealized molecular weights for the components. Both mono-phosphate ester and di-phosphate ester components include polyether phosphate ester and mono-alcohol phosphate ester. The polyether species include single end-capped polyether and fully end-capped polyether. It is understood that the fully end-capped polyether is present in the range of approximately 6-10 wt%.

### Example 4

To demonstrate the improved emulsifier properties of Product 1 from Example 1, oil-in-water emulsions were made using Product 1 and compared with a comparative phosphate ester emulsifier which was Crodafos CES available from Croda (INCI: Cetearyl Alcohol and Dicetyl Phosphate and Ceteth-10 Phosphate). Crodafos CES is a polyether phosphate ester produced using Ceteth-10 which is cetyl alcohol reacted with 10 mols of ethylene oxide. Therefore Crodafos CES comprises alkylene oxide residues. A Dino-Lite Edge 700-900x 5MP digital microscope was used as described in the Test Methods to produce the microscope images shown in FIGS. 1 to 6. From these images the particle size of the oil micelles created by each emulsifier for the non-polar oil, medium polarity oil and high polarity oil formulations can be compared. The specific formulations used are shown in Tables 2, 3 and 4. The formulations used to make each emulsion were adjusted to use 1 wt% of active product (from Product 1 or Crodafos CES) in each formulation.

**Table 2 - mineral oil emulsions - non-polar oil**

| Component | **Formulation of the Invention (wt%)** | | **Comparative Formulation (wt%)** | |
|---|---|---|---|---|
| **Part A** | | | | |
| Water | | 84.75 | | 84.75 |
| 10% NaOH solution | | 0.25 | | 0.25 |

| **Part B** | | | | |
|---|---|---|---|---|
| Product 1 | | 1.33 | | - |
| Crodafos CES | | - | | 4.00 |
| Mineral Oil | | 10.00 | | 10.00 |
| Stearyl alcohol | | 2.67 | | - |

| **Part C** | | | | |
|---|---|---|---|---|
| Euxyl PE9010 (Phenoxyethanol (and) Ethylhexylglycerin) | | 1.00 | | 1.00 |

**Table 3 - isopropyl myristate emulsions - medium polarity oil**

| Component | **Formulation of Invention (wt%)** | | **Comparative Formulation (wt%)** | |
|---|---|---|---|---|
| **Part A** | | | | |
| Water | | 84.75 | | 84.75 |
| 10% NaOH solution | | 0.25 | | 0.25 |

| **Part B** | | | | |
|---|---|---|---|---|
| Product 1 | | 1.33 | | - |
| Crodafos CES | | - | | 4.00 |
| Isopropyl Myristate Oil | | 10.00 | | 10.00 |
| Stearyl alcohol | | 2.67 | | - |

| **Part C** | | | | |
|---|---|---|---|---|
| Euxyl PE9010 (Phenoxyethanol (and) Ethylhexylglycerin) | | 1.00 | | 1.00 |

**Table 4 - di-isopropyl adipate emulsions - high polarity oil**

| Component | **Formulation of Invention (wt%)** | | **Comparative Formulation (wt%)** | |
|---|---|---|---|---|
| **Part A** | | | | |
| Water | | 84.75 | | 84.75 |
| 10% NaOH solution | | 0.25 | | 0.25 |

| **Part B** | | | | |
|---|---|---|---|---|
| Product 1 | | 1.33 | | - |
| Crodafos CES | | - | | 4.00 |
| Diisopropyl Adipate Oil | | 10.00 | | 10.00 |
| Stearyl alcohol | | 2.67 | | - |

| **Part C** | | | | |
|---|---|---|---|---|
| Euxyl PE9010 (Phenoxyethanol (and) Ethylhexylglycerin) | | 1.00 | | 1.00 |

*Emulsion Formulation Procedure:* Heat Part A ingredients in main beaker to 75 °C while mixing with a propeller blade at 360 rpm. In a separate beaker, heat Part B ingredients to 75 °C. When both phases are at temperature, add Part B to Part A. Allow to mix at temperature for 5 minutes and then begin cooling. Once temperature reaches ~ 46 °C, switch to a side sweep blade and lower mixing speed to 30 rpm. Add Part C under 40 °C. Adjust pH between 5-6 with 10% NaOH solution. All of the emulsions were made on the same day, and were all made using the above procedure.

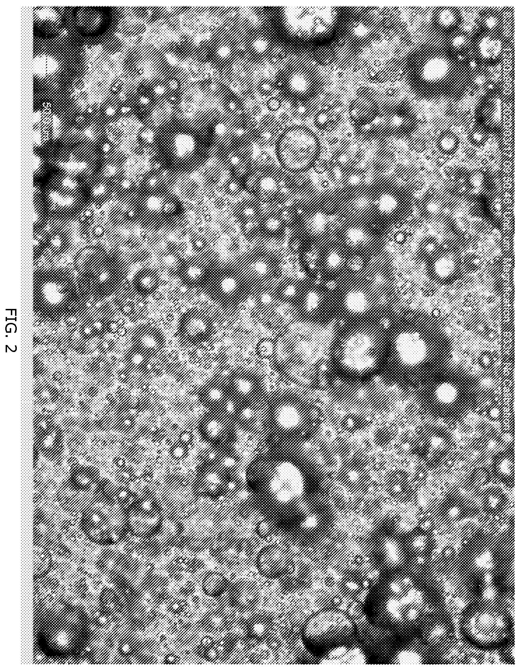

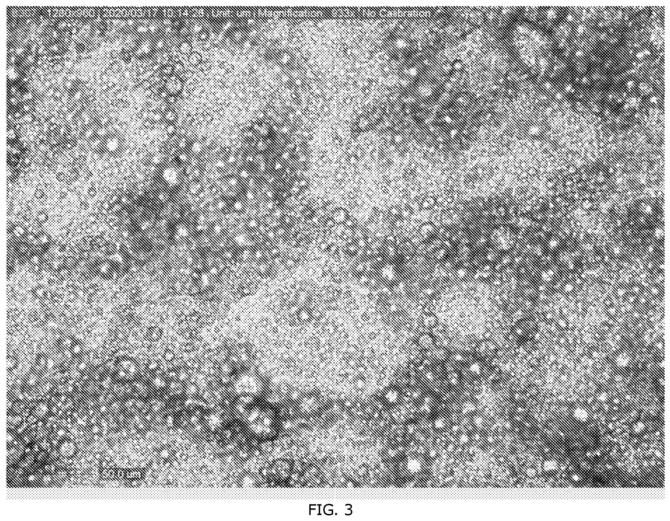

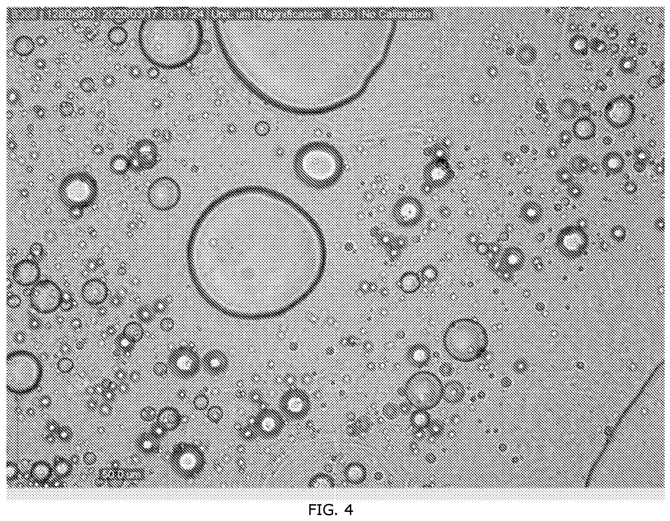

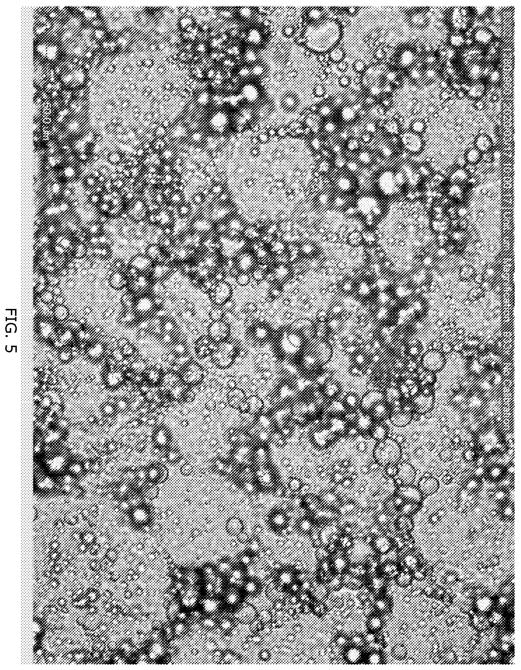

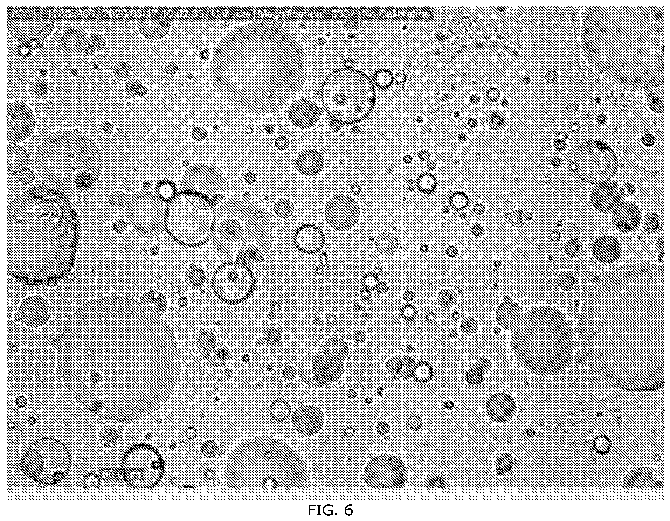

The microscope images shown in FIGS. 1 to 6 were taken as described in the Test Methods. It can be seen from comparing the emulsions shown in FIG. 1 (using Product 1) and FIG. 2 (Crodafos CES) that for the mineral oil (non-polar oil) in water emulsions shown in Table 2, Product 1 created oil micelle particles that were much smaller and more tightly packed than Crodafos CES. In contrast, the micelles created by Crodafos CES were less uniform in size, and the emulsion appeared to contain more air bubbles as well. It can be seen from FIG. 3 (Product 1) and FIG. 4 (Crodafos CES) that for the isopropyl myristate (medium polarity oil) in water emulsion, Product 1 created tightly packed oil micelle particles that had a visually small particle size. The emulsion containing Crodafos CES had multiple air bubbles and non-uniform particles, and the particles were spread apart and not packed together. It can be seen from FIG. 5 (Product 1) and FIG. 6 (Crodafos CES) that for the di-isopropyl adipate (high polarity oil) in water emulsion, Product 1 created oil micelle particles that were slightly varying in size, but were still tightly packed. Crodafos CES created bigger particles that were not uniform and that were spread apart. Thus it can be seen from FIGS. 1 to 6 that Product 1 produces emulsions with a visually smaller and more uniform particle size than Crodafos CES. Without being bound by theory, this may advantageously improve emulsion stability or homogeneity.

The particle size distributions of the mineral oil droplets (non-polar oil) in the emulsion formulations shown in Table 2 were also measured as described in the Test Methods above and the results are shown in FIG. 7 (Product 1) and FIG. 8 (Crodafos CES). The shape of the distribution peaks show a significant difference to the left (smaller) side with FIG. 7 showing that Product 1 produces a significant amount of particles with diameter under 10 µm in the emulsion while FIG. 8 shows no detection of particles with diameter under 10 µm. This is reflected in the D(v,0.1) value calculated for the distributions in FIGS. 7 and 8 which is the equivalent spherical diameter which 10% of all the particles (on a volume basis) fall below. The D(v,0.1) value was calculated as 11.2 µm for Product 1 and 26.9 µm for Crodafos CES which indicates that Product 1 produces more small particles in the emulsion. This is supported by comparing with the images in FIGS. 1 and 2.

### Example 5

The following formulations are provided to indicate different ways in which Product 1 may be used.

**Table 5: Skin Care Formulation**

| Ingredient | | | %w/w |
|---|---|---|---|
| **Part A** | | | |
| Water | | | 65.54 |
| Glycerin | | | 8.00 |
| Xanthan Gum | | | 0.20 |
| Sodium hydroxide (25% solution) | | | 1.26 |

| **Part B** | | | |
|---|---|---|---|
| Product 1 | | | 5.00 |
| Crodacol 1618 (Cetearyl Alcohol) | | | 3.00 |
| Crodamol GTCC (Caprylic/Capric Triglyceride) | | | 5.00 |
| Crodamol OSU (Diethylhexyl Succinate) | | | 5.00 |
| Crodamol GTEH (Triethylhexanoin) | | | 5.00 |
| Sensasil PCA (PCA Dimethicone) | | | 1.00 |

| **Part C** | | | |
|---|---|---|---|
| Euxyl PE9010 (Phenoxyethanol (and) Ethylhexylglycerin) | | | 1.00 |
| | **Total** | | 100.00 |

### Procedure:

In main beaker, weigh the water and 25% sodium hydroxide solution and mix. In a weigh boat combine glycerin and xanthan gum and make a slurry and add to the water. Heat Part A to 75 °C while mixing. In a separate beaker, combine Part B ingredients and heat to 75 °C. Add Part B to Part A once both are at 75 °C and mix for 10 minutes. Cool to 40 °C and add Part C. Adjust the pH to 6-6.5 if necessary.

**Table 6: Low pH Skin Care Formulation**

| Ingredient | %w/w |
|---|---|
| **Part A** | |
| Water | 70.00 |
| AHA Concentrate OG | 3.00 |
| Sodium citrate | 1.00 |
| Glycerin | 2.00 |
| Zemea (Propanediol) | 2.00 |
| Xanthan Gum | 0.20 |
| Disodium EDTA | 0.30 |

| **Part B** | |
|---|---|
| Product 1 | 2.00 |
| Crodacol 1618 (Cetearyl Alcohol)) | 3.00 |
| Crodamol GTCC (Caprylic/Capric Triglyceride) | 5.00 |
| Crodamol STS (PPG-3 Benzyl Ether Myristate) | 5.00 |
| Crodamol IPIS (Isopropyl Isostearate) | 5.00 |

| **Part C** | |
|---|---|
| Euxyl PE9010 (Phenoxyethanol (and) Ethylhexylglycerin) | 1.00 |
| **Total** | 100.00 |

### Procedure:

In main beaker, add water with AHA, citric acid and sodium citrate and mix until everything is completely dissolved and uniform. Combine glycerin, Zemea and xanthan gum to make a slurry and add to the acid mixture. Add remaining ingredients and heat to 70 °C. In a separate beaker, combine Part B ingredients and heat to 70 °C and mix. Add Part B into Part A while mixing and mix for 10 minutes while maintaining the temperature. Start cooling with propeller blade to 55-60 °C and switch to side sweep at 60 °C. Cool 40 °C and add Part C. Check pH and ensure that it is between 3.5-4.0.

**Table 7: Organic Sunscreen**

| Ingredient | %w/w |
|---|---|
| **Part A** | |
| Water | 50.60 |
| Glycerin | 4.00 |
| Sodium Hydroxide (20% solution) | 0.25 |
| Disodium EDTA | 0.10 |

| **Part B** | |
|---|---|
| Product 1 | 1.38 |

| **Part C** | |
|---|---|
| Crodacol S95 (Stearyl Alcohol) | 4.88 |
| Cromollient ESP (Tris (PPG-3 Benzyl) Citrate) | 5.00 |
| Crodamol SFX (PPG-3 Benzyl Ether) Ethylhexanoate) | 5.00 |
| Homosalate | 10.00 |
| Octisalate | 5.00 |
| Octocrylene | 2.80 |
| Oxybenzone | 6.00 |
| Avobenzone | 3.00 |

| **Part D** | |
|---|---|
| ViscOptima SE (Sodium Polyacrylate (and) Ethylhexyl Cocoate (and) PPG-3 Benzyl Ether Myristate (and) Polysorbate 20) | 1.00 |
| Euxyl PE9010 (Phenoxyethanol (and) Ethylhexylglycerin) | 1.00 |
| Total | 100.00 |

### Procedure:

Combine Part A ingredients and heat to 75-80 °C while mixing with overhead mixer. In separate beaker heat Part B to 75 °C. Add Part B into Part A and mix at 75 °C for 10 minutes or until homogenous. In separate beaker, combine Part C ingredients and heat to 75 °C while mixing. Add Part C into Part A/B and homogenize for 3-5 minutes or until uniform. Cool the batch with side sweep to 45 °C and add Part D. Continue mixing batch until cools to room temperature.

**Table 8: Sunscreen with organic and inorganic sun filters**

| Ingredient | %w/w |
|---|---|
| **Part A** | |
| Water | 58.45 |
| Glycerin | 4.00 |
| Xanthan gum | 0.20 |
| Sodium Hydroxide (20% solution) | 0.25 |
| Disodium EDTA | 0.10 |

| **Part B** | |
|---|---|
| Product 1 | 2.00 |

| **Part C** | |
|---|---|
| Crodacol 1618 (Cetearyl Alcohol) | 3.00 |
| Crodamol ISIS (Isostearyl Isostearate) | 5.00 |
| Crodamol SFX (PPG-3 Benzyl Ether) | 5.00 |
| Solaveil HTP-1 (Titanium Dioxide (and) Alumina (and) Stearic Acid) | 15.00 |
| Avobenzone | 3.00 |

| **Part D** | |
|---|---|
| SolPerform 100 (Aqua (and) Hydrolyzed Wheat Protein/PVPCrosspolymer) | 3.00 |
| Euxyl PE9010 (Phenoxyethanol (and) Ethylhexylglycerin) | 1.00 |
| Total | 100.00 |

### Procedure:

Combine Part A ingredients and heat to 75-80 °C while mixing with overhead mixer. In separate beaker heat Part B to 75 °C. Add Part B into Part A and mix at 75 °C for 10 minutes or until homogenous. In separate beaker, combine Part C ingredients and heat to 75 °C while mixing. Add Part C into Part A/B and homogenize for 3-5 minutes or until uniform. Cool the batch with side sweep to 45 °C and add Part D. Continue mixing until temperature batch cools to room temperature.

**Table 9: Hair Conditioner/Hair treatments**

| Ingredient | %w/w |
|---|---|
| **Part A** | |
| Water | 83.88 |
| Sodium Hydroxide (10% solution) | 0.62 |

| **Part B** | |
|---|---|
| Crodamol STS (PPG-3 Benzyl Ether Myristate) | 10.00 |
| Crodacol 1618 (Cetearyl Alcohol) | 2.50 |
| Product 1 | 2.00 |

| **Part C** | |
|---|---|
| Euxyl PE9010 (Phenoxyethanol (and) Ethylhexylglycerin) | 1.00 |
| Total | 100.00 |

### Procedure:

Add water to the main beaker and add NaOH solution to pH 4 and heat Part A to 75 °C. Combine Part B ingredients and heat to 75 °C or completely melted. Add Part B into Part A and continue mixing at low to moderate speed while cooling. Check the pH and adjust if needed.

**Table 10: Hair Colour Cream**

| Ingredients | %w/w |
|---|---|
| **Part A** | |
| Crodacol C95 (Cetyl Alcohol) | 5.67 |
| Crodamol IPP (Isopropyl Palmitate) | 5.00 |
| Product 1 | 2.73 |
| Paraffin wax | 1.00 |
| Incroquat Behenyl TMC-85 (Behentrimonium Chloride (and) Isopropyl Alcohol) | 0.90 |

| **Part B** | |
|---|---|
| Deionized Water | 59.89 |
| Tetrasodium EDTA | 0.40 |
| Sodium Sulfite | 0.40 |
| Ascorbic Acid | 0.20 |
| p-Phenylendiamine | 0.30 |
| p-Aminophenol | 0.14 |
| 2-Methyl-5-Hydroxyethylaminophenol | 0.09 |
| 4-Amino-2-Hydroxytoluene | 0.07 |
| Resorcinol | 0.06 |
| 2-Amino-3-Hydroxypyridine | 0.17 |
| m-Aminophenol | 0.02 |
| Monoethanolamine | 0.10 |
| Deionized Water | 4.25 |

| **Part C** | |
|---|---|
| Ammonium Hydroxide 28% | 6.50 |
| Ammonium Bicarbonate | 2.00 |
| Deionized Water | 10.11 |
| Total | 100.00 |

### Procedure:

Combine the first 5 ingredients of Part B and heat to 80-85 °C, mixing moderately until all is dissolved. Add dye intermediates and mix for 20 minutes until completely dissolved. In a separate beaker, heat and mix Part A to 80-85 °C. Add Part A to Part B and mix for 20 minutes. Cool to 40 °C before adding Part D and continue mixing for another 10 minutes.

**Table 11: 20 Volume Cream Developer for Cream Colorant**

| Ingredient/INCI Name | % w/w |
|---|---|
| **Part A** | |
| Water Deionised (Aqua) | 75.70 |
| Disodium EDTA | 0.20 |
| Sodium Stannate | 0.10 |
| Product 1 | 6.00 |
| Glycerin | 1.00 |

| **Part B** | |
|---|---|
| Hydrogen Peroxide (35% Cosmetic Grade) | 17.00 |

### Procedure:

Heat water in main vessel and add the next two ingredients of Part A, with moderate mixing until completely dissolved. Continue mixing and add remaining ingredients of Part A, one after the other, and heat to 70-75 °C. Mix for 10 minutes, and then cool to 40 °C. Add Part B and mix for 15 minutes. Adjust pH to 2.5-3.5.

**Table 12: Anti-Perspirant**

| Ingredients | Weight % |
|---|---|
| **Part A** | |
| Crodamol STS | 47.00 |
| ARLAMOL^{™} PM3 (PPG-3 Myristyl Ether) | 3.00 |
| ARLAMOL PB14 (PPG-14 Butyl Ether) | 2.00 |
| ARLAMOL PC10 (PPG-10 Cetyl Ether) | 2.00 |
| Product 1 | 1.00 |

| **Part B** | |
|---|---|
| CRODACOL^{™} S95 (Stearyl Alcohol) | 16.00 |
| Hydrogenated Castor Oil | 3.50 |
| Corn Starch Modified | 3.00 |
| Fumed Silica | 0.50 |

| **Part C** | |
|---|---|
| Aluminum Zirconium Tetrachlorohydrex GLY | 22.00 |

### Procedure:

Combine Part A ingredients with mixing and heat to 80-85 °C. Add first two ingredients of Part B. Once dissolved, add remaining ingredients of Part B one at a time. Stir until uniform and cool to 60-65 °C. Maintain this temperature and add Part C, mixing well. Cool to 55 °C with mixing and pour into molds.

**Table 13: Tinted Moisturizer Cream**

| Ingredients | %w/w |
|---|---|
| **Part A** | |
| Water | To 100 |
| Glycerin | 3.00 |
| Magnesium Aluminum Silicate | 1.00 |
| Xanthan Gum | 0.50 |
| 1, 3 Propanediol | 4.00 |
| Solaveil XT-40W (Titanium Dioxide (and) Water (and) Polyglyceryl-2 Caprate (and) Sucrose Stearate (and) Simmondsia | 15.00 |
| Chinensis (Jojoba) Seed Oil (and) Stearic Acid (and) Alumina (and) Glyceryl Caprylate (and) Squalane) | |

| **Part B** | |
|---|---|
| SP Crodamol^{™} GTCC MBAL (Caprylic/Capric Triglyceride) | 11.75 |
| Crodamol ISIS (Isostearyl Isostearate) | 3.00 |
| Crodamol SSA (Decyl Isostearate (and) Isostearyl Isostearate) | 3.00 |
| Product 1 | 3.00 |

| **Part C** | |
|---|---|
| SP Crodamol GTCC MBAL (Caprylic/Capric Triglyceride) | 1.25 |
| Iron Oxide (and) CI77492 | 0.96 |
| Iron Oxide (and) CI77491 | 0.24 |
| Iron Oxide (and) CI77499 | 0.06 |

| **Part D** | |
|---|---|
| Lactic Acid | qs |
| Sodium Benzoate (and) Potassium Sorbate | 1.50 |

| **Part E** | |
|---|---|
| Venuceane (Thermus Thermophilus Ferment (and) Glycerin) | 3.00 |

### Procedure:

Combine Part C ingredients, weigh out 210% more for each ingredient. Mix thoroughly, then pass through the three-roller mill 3x at setting 3 (loosest setting). Combine Part B ingredients and heat with mixing to 70 °C. Add Part C into Part B while mixing. For Part A, add magnesium aluminum silicate to water and heat with mixing to 70 °C. Combine glycerin and xanthan gum together to make a slurry and then add to Part A. When the temperature is at 70 °C, add Solaveil XT-40W to Part A. Maintain temperature and mix until homogenous. With strong mixing, add Part B/C into Part A; mix for 5 minutes. Place batch under homogenizer and mill for 3 minutes. Cool to 45 °C with stirring. Adjust the pH with lactic acid to 4.10 while stirring and add the Sodium Benzoate (and) Potassium Sorbate preservative (Part D). Add the Venuceane (Part E) and mix until homogenous.

**Table 14 - Inorganic UV filter Sunscreen**

| **Ingredient** | **%w/w** |
|---|---|
| **Part A** | |
| Water | 42.95 |
| Pricerine 9091 (Glycerin) | 5.00 |
| Veegum Ultra (Magnesium Aluminum Silicate) | 0.80 |
| Keltrol CG (Xanthan gum) | 0.20 |
| Disodium EDTA | 0.05 |

| **Part B** | |
|---|---|
| Product 1 | 1.33 |
| Crodacol 1618 | 2.67 |
| Crodamol CAP (Cetearyl Ethylhexanoate (and) Isopropyl Myristate) | 5.00 |
| Crodamol ISIS (Isostearyl Isostearate) | 3.00 |
| Crodamol IPM (Isopropyl Myristate) | 4.00 |
| Crodamol GTCC (Caprylic/Capric Triglyceride) | 6.00 |
| Solaveil XT-300 | 28.00 |

| **Part C** | |
|---|---|
| Euxyl PE9010 | 1.00 |

### Procedure:

Premix the xanthan gum, veegum ultra, and glycerin. Add water and EDTA with stirring and heat to 70-75 °C. Combine all Part B ingredients except Solaveil XT-300 and heat 70-75 °C. Add Solaveil XT-300 to Part B with stirring and reheat to 70-75 °C. Add Part B to Part A with stirring and homogenise for two minutes. Cool to 40 °C with a side-sweep blade and add Part C. Adjust the pH if necessary.

### Example 6

The SPF (sun protection factor) performance of Product 1 from Example 1 was compared with Crodafos CES available from Croda. The in-vitro SPF performance of an inorganic sunscreen formulation comprising Product 1 or Crodafos CES was tested as described in the Test Methods. Formulations A & B shown in Table 15 were adjusted to use 1 wt% of active product (from Product 1 or Crodafos CES) in each formulation.

**Table 15: Inorganic Sunscreen Formulations**

| **Ingredient** | **Formulation A (%w/w)** | **Formulation B (%w/w)** |
|---|---|---|
| **Part A** | | |
| Water | 39.89 | 39.89 |
| Pricerine 9091 (Glycerin) | 8.00 | 8.00 |
| Veegum Ultra (Magnesium Aluminum Silicate) | 0.80 | 0.80 |
| Keltrol CG (Xanthan gum) | 0.20 | 0.20 |
| Disodium EDTA | 0.05 | 0.05 |
| Sodium hydroxide 10%solution | 0.060 | 0.060 |

| **Part B** | | |
|---|---|---|
| Product 1 | 1.33 | 0.00 |
| Crodafos CES (Cetearyl Alcohol (and) Dicetyl Phosphate (and) Ceteth -10 Phosphate) | 0.00 | 4.00 |
| Crodacol 1618 (Cetearyl Alcohol) | 2.67 | 0.00 |
| Crodamol CAP (Cetearyl Ethylhexanoate (and) Isopropyl Myristate) | 5.00 | 5.00 |
| Crodamol ISIS (Isostearyl Isostearate) | 3.00 | 3.00 |
| Crodamol IPM (Isopropyl Myristate) | 4.00 | 4.00 |
| Crodamol GTCC (Caprylic/Capric Triglyceride) | 6.00 | 6.00 |
| Solaveil XT-300 (Titanium Dioxide (and) Caprylic/Capric Triglyceride (and) Polyhydroxystearic Acid (and) Stearic Acid (and) Alumina) | 28.00 | 28.00 |

| **Part C** | | |
|---|---|---|
| Euxyl PE9010 (Phenoxyethanol (and) Ethylhexylglycerin) | 1.00 | 1.00 |
| Total | 100.00 | 100.00 |

Formulations A & B from Table 15 were tested for in-vitro SPF performance as described in the Test Methods. The results are given in Table 16.

**Table 16: In-Vitro SPF results**

| | **Formulation A** | | **Formulation B** |
|---|---|---|---|
| | **Batch 1** | **Batch 2** | **Batch 1** |
| **Before Radiation** | | | |
| Mean SPF | 38.11 | 46.55 | 24.06 |

| **After Radiation** | | | |
|---|---|---|---|
| Mean SPF | 38.28 | 43.27 | 22.313 |
| Critical Wavelength | 380.00 | 380.00 | 380.73 |

It can be seen from Table 16 that Product 1 in Formulation A provided a significant improvement of In-Vitro SPF performance (higher values) when compared with Crodafos CES in an equivalent Formulation B. This improvement was seen in two batches of Formulation A both before and after exposure to UV radiation.

### Example 7

The ability of Product 1 from Example 1 to improve the delivery and/or transfer of the active skincare ingredient caffeine to the skin from a formulation was compared with Crodafos CES and a combination of known emulsifiers (Arlacel 2121 & Cithrol DPHS) available from Croda. The active delivery was tested as described in the Test Methods. Formulations A, B & C shown in Table 17 were adjusted to include 1 wt% of active product in each formulation.

**Table 17: Actives Delivery Formulation with 2% Caffeine**

| **Ingredient** | **Formulation A (%w/w)** | **Formulation B (%w/w)** | **Formulation C (%w/w)** |
|---|---|---|---|
| **Part A** | | | |
| Water | 81.12 | 80.12 | 81.75 |
| Glycerin | 2.00 | 2.00 | 2.00 |
| Xanthan gum | 0.25 | 0.25 | 0.25 |
| Caffeine | 2.00 | 2.00 | 2.00 |
| Sodium Hydroxide (10% solution) | 0.63 | 0.63 | 0.00 |

| **Part B** | | | |
|---|---|---|---|
| Mineral Oil | 10.00 | 10.00 | 10.00 |
| Crodacol 1618 (Cetearyl Alcohol) | 1.67 | 0.00 | 2.00 |
| Product 1 | 1.33 | 0.00 | 0.00 |
| Crodafos CES (Cetearyl Alcohol (and) Dicetyl Phosphate (and) Ceteth - 10 Phosphate) | 0.00 | 4.00 | 0.000 |
| Arlacel 2121 (Sorbitan Stearate (and) Sucrose Cocoate) | 0.00 | 0.00 | 0.853 |
| Cithrol DPHS (PEG 30 Dipolyhydroxystearate) | 0.00 | 0.00 | 0.143 |

| **Part C** | | | |
|---|---|---|---|
| Euxyl PE9010 (Phenoxyethanol (and) Ethylhexylglycerin) | 1.00 | 1.00 | 1.00 |
| Total | 100.00 | 100.00 | 100.00 |

Formulations A, B & C from Table 17 were tested for actives delivery performance as described in the Test Methods. The results are given in Table 18.

**Table 18: Actives Delivery of Caffeine**

| **Formulation** | **wt% of Caffeine Transferred** |
|---|---|
| A | 21.70% |
| B | 16.80% |
| C | 19.60% |

It can be seen from Table 18 that Product 1 in Formulation A provided an improvement in the amount of active ingredient (caffeine) transferred when compared with both Formulations B & C.

It is to be understood that the invention is not to be limited to the details of the above embodiments, which are described by way of example only. Many variations are possible.

## Claims

1. A composition which comprises:
i) a polyether phosphate ester which does not comprise an alkylene oxide residue;
ii) a mono-alcohol phosphate ester; and
iii) a polyether which comprises at least two terminal C6 to C36 hydrocarbyl groups.

2. A composition according to claim 1 wherein the hydrocarbyl groups of the polyether are alkyl or alkenyl groups.

3. A composition according to claim 1 or 2 wherein the polyether phosphate ester comprises at least one terminal C6 to C36, preferably C8 to C22, hydrocarbyl group.

4. A composition according to claim 3 wherein the hydrocarbyl group of the polyether phosphate ester is an alkyl or alkenyl group.

5. A composition according to any preceding claim wherein the polyether phosphate ester comprises 3 to 15 ether bonds.

6. A composition according to any preceding claim wherein the polyether phosphate ester does not comprise a carboxylic acid ester bond.

7. A composition according to any preceding claim wherein the polyether phosphate ester is obtainable by reacting a first polyether with a phosphorus oxide, preferably with phosphorous pentoxide.

8. A composition according to claim 7 wherein the first polyether is the reaction product of reactants comprising a diol and a mono-alcohol.

9. A composition according to claim 8 wherein the diol comprises 2 to 6 carbon atoms.

10. A composition according to claim 8 or 9 wherein the mono-alcohol comprises 12 to 20 carbon atoms.

11. A compound according to any of claims 8 to 10 wherein the molar ratio of diol to mono-alcohol is from 2:1 to 20:1, preferably from 3:1 to 12:1, particularly from 4:1 to 10:1.

12. A composition according to any of claims 1 to 11 wherein the amount of the polyether phosphate ester in the composition is at least 20 wt% on the basis of the total weight of the composition.

13. A composition according to any of claims 1 to 12 wherein the amount of the mono-alcohol phosphate ester in the composition is at least 10 wt% on the basis of the total weight of the composition.

14. A composition according to any of claims 1 to 13 wherein the amount of the polyether comprising at least two terminal C6 to C36 hydrocarbyl groups in the composition is at least 2 wt% on the basis of the total weight of the composition.

15. An emulsion comprising the composition according to any of claims 1 to 14.

16. A personal care formulation comprising a compound which is a polyether phosphate ester and which does not comprise an alkylene oxide residue.

17. A personal care formulation comprising the composition according to any of claims 1 to 14.

18. A personal care formulation according to claim 16 or 17 further comprising a sunfilter or sunscreen material.

19. Use of the composition according to any of claims 1 to 14 as a surfactant.

20. Use of the composition according to any of claims 1 to 14, to reduce the D(v,0.1) particle size of an emulsion, preferably measured as described herein.

21. A method of forming an emulsion in which:
a) the composition according to any of claims 1 to 14;
b) a first phase component; and
c) a second phase component;
are combined in any order, including simultaneously.

22. The method of claim 21 wherein the emulsion is a water-in-oil-in-water (W/O/W) emulsion.

## Patentansprüche

1. Zusammensetzung umfassend:
i) einen Polyetherphosphatester, der keinen Alkylenoxidrest umfasst;
ii) einen Monoalkoholphosphatester; und
iii) einen Polyether, der wenigstens zwei endständige C6-bis C36-Hydrocarbylgruppen umfasst.

2. Zusammensetzung nach Anspruch 1, wobei die Hydrocarbylgruppen des Polyethers Alkyl- oder Alkenylgruppen sind.

3. Zusammensetzung nach Anspruch 1 oder 2, wobei der Polyetherphosphatester wenigstens eine endständige C6-bis C36-, vorzugsweise C8- bis C22-, Hydrocarbylgruppe umfasst.

4. Zusammensetzung nach Anspruch 3, wobei die Hydrocarbylgruppe des Polyetherphosphatesters eine Alkyl- oder Alkenylgruppe ist.

5. Zusammensetzung nach einem der vorstehenden Ansprüche, wobei der Polyetherphosphatester 3 bis 15 Etherbindungen umfasst.

6. Zusammensetzung nach einem der vorstehenden Ansprüche, wobei der Polyetherphosphatester keine Carbonsäureesterbindung umfasst.

7. Zusammensetzung nach einem der vorstehenden Ansprüche, wobei der Polyetherphosphatester durch Umsetzen eines ersten Polyethers mit einem Phosphoroxid, vorzugsweise mit Phosphorpentoxid, erhältlich ist.

8. Zusammensetzung nach Anspruch 7, wobei der erste Polyether das Reaktionsprodukt von Reaktanten ist, die ein Diol und einen Monoalkohol umfassen.

9. Zusammensetzung nach Anspruch 8, wobei das Diol 2 bis 6 Kohlenstoffatome umfasst.

10. Zusammensetzung nach Anspruch 8 oder 9, wobei der Monoalkohol 12 bis 20 Kohlenstoffatome umfasst.

11. Verbindung nach einem der Ansprüche 8 bis 10, wobei das Molverhältnis von Diol zu Monoalkohol von 2:1 bis 20:1, vorzugsweise von 3:1 bis 12:1, insbesondere von 4:1 bis 10:1, beträgt.

12. Zusammensetzung nach einem der Ansprüche 1 bis 11, wobei die Menge des Polyetherphosphatesters in der Zusammensetzung wenigstens 20 Gew.-% auf der Basis des Gesamtgewichts der Zusammensetzung beträgt.

13. Zusammensetzung nach einem der Ansprüche 1 bis 12, wobei die Menge des Monoalkoholphosphatesters in der Zusammensetzung wenigstens 10 Gew.-% auf der Basis des Gesamtgewichts der Zusammensetzung beträgt.

14. Zusammensetzung nach einem der Ansprüche 1 bis 13, wobei die Menge des Polyethers, der wenigstens zwei endständige C6- bis C36-Hydrocarbylgruppen umfasst, in der Zusammensetzung wenigstens 2 Gew.-% auf der Basis des Gesamtgewichts der Zusammensetzung beträgt.

15. Emulsion umfassend die Zusammensetzung nach einem der Ansprüche 1 bis 14.

16. Körperpflegeformulierung umfassend eine Verbindung, die ein Polyetherphosphatester ist und die keinen Alkylenoxidrest umfasst.

17. Körperpflegeformulierung umfassend die Zusammensetzung nach einem der Ansprüche 1 bis 14.

18. Körperpflegeformulierung nach Anspruch 16 oder 17, ferner umfassend ein Sonnenfilter- oder Sonnenschutzmaterial.

19. Verwendung der Zusammensetzung nach einem der Ansprüche 1 bis 14 als Tensid.

20. Verwendung der Zusammensetzung nach einem der Ansprüche 1 bis 14, um die D(V,0,1)-Partikelgröße einer Emulsion, vorzugsweise gemessen wie hier beschrieben, zu verringern.

21. Verfahren zum Bilden einer Emulsion, wobei:
a) die Zusammensetzung nach einem der Ansprüche 1 bis 14;
b) eine erste-Phase-Komponente; und
c) eine zweite-Phase-Komponente;
in beliebiger Reihenfolge, einschließlich gleichzeitig, kombiniert werden.

22. Verfahren nach Anspruch 21, wobei die Emulsion eine Wasser-in-Öl-in-Wasser(W/O/W)-Emulsion ist.

## Revendications

1. Composition qui comprend :
i) un ester de phosphate de polyéther ne comprenant pas un radical oxyde d'alkylène ;
ii) un ester de phosphate de mono-alcool ; et
iii) un polyéther qui comprend au moins deux groupes hydrocarbyle terminaux en C6 à C36.

2. Composition selon la revendication 1, dans laquelle les groupes hydrocarbyle du polyéther sont des groupes alkyle ou alcényle.

3. Composition selon la revendication 1 ou 2, dans laquelle l'ester de phosphate de polyéther comprend au moins un groupe hydrocarbyle terminal en C6 à C36, de préférence en C8 à C22.

4. Composition selon la revendication 3, dans laquelle le groupe hydrocarbyle de l'ester de phosphate de polyéther est un groupe alkyle ou alcényle.

5. Composition selon l'une quelconque des revendications précédentes, dans laquelle l'ester de phosphate de polyéther comprend 3 à 15 liaisons éther.

6. Composition selon l'une quelconque des revendications précédentes, dans laquelle l'ester de phosphate de polyéther ne comprend pas une liaison ester d'acide carboxylique.

7. Composition selon l'une quelconque des revendications précédentes, dans laquelle l'ester de phosphate de polyéther peut être obtenu par mise en réaction d'un premier polyéther avec un oxyde de phosphore, de préférence avec du pentoxyde de phosphore.

8. Composition selon la revendication 7, dans laquelle le premier polyéther est le produit de réaction de réactifs comprenant un diol et un mono-alcool.

9. Composition selon la revendication 8, dans laquelle le diol comprend 2 à 6 atomes de carbone.

10. Composition selon la revendication 8 ou 9, dans laquelle le mono-alcool comprend 12 à 20 atomes de carbone.

11. Composé selon l'une quelconque des revendications 8 à 10, dans lequel le rapport molaire de diol sur mono-alcool est de 2:1 à 20:1, de préférence de 3:1 à 12:1, en particulier de 4:1 à 10:1.

12. Composition selon l'une quelconque des revendications 1 à 11, dans laquelle la quantité de l'ester de phosphate de polyéther dans la composition est d'au moins 20 % en poids sur la base du poids total de la composition.

13. Composition selon l'une quelconque des revendications 1 à 12, dans laquelle la quantité de l'ester de phosphate de mono-alcool dans la composition est d'au moins 10 % en poids sur la base du poids total de la composition.

14. Composition selon l'une quelconque des revendications 1 à 13, dans laquelle la quantité du polyéther comprenant au moins deux groupes hydrocarbyle terminaux en C6 à C36 dans la composition est d'au moins 2 % en poids par rapport au poids total de la composition.

15. Émulsion comprenant la composition selon l'une quelconque des revendications 1 à 14.

16. Formulation de soins personnels comprenant un composé qui est un ester de phosphate de polyéther et qui ne comprend pas de radical oxyde d'alkylène.

17. Formulation de soins personnels comprenant la composition selon l'une quelconque des revendication 1 à 14.

18. Formulation de soins personnels selon la revendication 16 ou 17, comprenant en outre un filtre solaire ou une matière écran solaire.

19. Utilisation de la composition selon l'une quelconque des revendications 1 à 14 en tant qu'agent tensioactif.

20. Utilisation de la composition selon l'une quelconque des revendications 1 à 14, pour réduire la taille de particule D(v, 0,1) d'une émulsion, de préférence mesurée comme décrit ici.

21. Procédé de formation d'une émulsion dans lequel :
a) la composition selon l'une quelconque des revendications 1 à 14 ;
b) un premier composant de phase ; et
c) un deuxième composant de phase ;
sont combinés dans n'importe quel ordre, y compris simultanément.

22. Procédé selon la revendication 21, dans lequel l'émulsion est une émulsion eau-dans-huile-dans-eau (E/H/E) .
